# EUROPEAN PATENT SPECIFICATION

(11) **EP 3 848 357 B1**
(45) Date of publication and mention of the grant of the patent: **18.01.2023**
(21) Application number: 20211201.7
(22) Date of filing: 02.12.2020
(51) Int. Cl.: C07D 249/20, C07D 403/14, C07D 405/14, C09D 7/48, G03C 1/815

(54) **REACTIVE BENZOTRIAZOLE UV ABSORBER AND USE THEREOF**
REAKTIVE BENZOTRIAZOL-UV-ABSORBER UND VERWENDUNG DAVON
ABSORBEUR REACTIVE D'UV DE TYPE BENZOTRIAZOLE ET SON UTILISATION

(30) Priority: 13.01.2020 TW 109101084
(43) Date of publication of application: 14.07.2021
(73) Proprietor: Everlight Chemical Industrial Corporation, 106 Taipei City (TW)
(72) Inventor: CHEN, Chih-Wei, 328 Taoyuan City (TW); CHEN, Ko-Lun, 328 Taoyuan City (TW); HUANG, Yao-Hsing, 328 Taoyuan City (TW)
(74) Representative: van Dam, Vincent

(56) References cited:
- EP-A1- 3 578 550
- US-A- 3 738 837

## Description

### CROSS REFERENCE TO RELATED APPLICATION

This application claims the benefits of the Taiwan Patent Application Serial Number 109101084, filed on January 13, 2020.

### BACKGROUND

### 1. Field

The present disclosure relates to a novel benzotriazole UV absorber with red shifts at the absorption peaks thereof and use thereof. In particular, the present disclosure relates to a novel benzotriazole UV absorber with excellent UV-light absorption property, a composition comprising the same, a glasses lens and a protection film.

### 2. Description of Related Art

An UV absorber is a light stabilizer, which can absorb the UV light in the sun light or fluorescent light. Thus, it is possible to prevent the material contained the UV absorber from being damaged by the UV light. Alternatively, when a coating layer containing the UV absorber is formed on a substrate, it is possible to prevent the substrate from being damaged by the UV light.

Currently, the known UV absorbers mainly can be classified into benzotriazole-based, methyl salicylate-based, benzophenone-based, substituted acrylonitrile-based and triazine-based UV absorbers. Among them, the benzotriazole-based UV absorbers are UV absorbers with high stability. Thus, the material or substrate containing the benzotriazole-based UV absorbers can be prevented from being damaged by the UV light.

However, the absorption ranges of the commercial available benzotriazole-based UV absorbers are still limited to about 400 nm. Thus, it is desirable to provide a novel benzotriazole-based UV absorber, wherein the absorption spectrum thereof shows red shifts at the absorption peaks, and thus the application of the benzotriazole-based UV absorbers can further be extended.

### SUMMARY

An object of the present disclosure is to provide a novel compound, which has excellent UV light absorption property and extraction resistance.

The compound provided by the present disclosure is represented by the following formula (I): 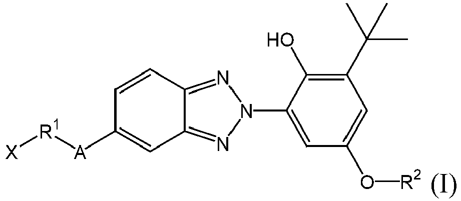 wherein,
A is -S- or -SO₂-;
R¹ is straight or branched C₁₋₁₀ alkylene;
R² is -R³Y;
R³ is straight or branched C₁₋₁₀ alkylene;
each of X and Y respectively is -OH or -OCOR⁴ and
R⁴ is straight or branched C₃₋₁₀ alkenyl.

The novel compound provided by the present disclosure is a benzotriazole compound, which can be used as an UV absorber. Herein, a sulfur-containing group is located at the 5-position on the benzo ring of the formula (I), and an oxygen-containing group is located at the 5-position on the phenyl group of the formula (I). By the synergy effect of the sulfur-containing group and the oxygen-containing group, the spectrum of the compound shows a red shift at the absorbance peaks, and thus the compound has an extended absorption range. In particular, the novel compound provided by the present disclosure has an absorption range extended to 450 nm or more, and also has the absorbance peaks at 280 nm to 340 nm which is the conventional absorbance peaks of the UV absorber. Thus, the compound of the present application can be applied to anti-blue light materials for a product such as lens, contacts, etc., and can further be applied to carbon fiber complex materials for aerospace and transportation. In addition, the novel compound of the present disclosure contains a reactive group, and can be polymerized with monomers or oligomers to form polymers with UV absorbers. The UV absorbers in the obtained polymer show excellent extraction resistance.

In the present disclosure, A is -S- or -SO₂-.

In the present disclosure, R¹ is straight or branched C₁₋₁₀ alkylene. In one embodiment of the present disclosure, R¹ can be straight or branched C₁₋₆ alkylene. In another embodiment of the present disclosure, R¹ can be straight or branched C₁₋₃ alkylene.

In the present disclosure, R² is -R³Y.

In the present disclosure, R³ is straight or branched C₁₋₁₀ alkylene. In one embodiment of the present disclosure, R³ can be straight or branched C₁₋₆ alkylene. In another embodiment of the present disclosure, R³ can be straight or branched C₁₋₃ alkylene.

In the present disclosure, each of X and Y respectively is -OH or -OCOR⁴, wherein R⁴ can be straight or branched C₃₋₁₀ alkenyl. In one embodiment of the present disclosure, each of X and Y respectively can be -OH or -OCOR⁴, whereinR⁴ can be straight or branched C₃₋₆ alkenyl. In another embodiment of the present disclosure, each of X and Y respectively can be -OH or -OCOR^{4a}, wherein R^{4a} can be straight or branched C₃₋₆ alkenyl.

In one embodiment of the present disclosure, A can be -S-; R¹ can be straight or branched C₁₋₁₀ alkylene; and X can be -OH. In another embodiment of the present disclosure, A can be -S-; R¹ can be straight or branched C₁₋₆ alkylene; and X can be -OH. In another embodiment of the present disclosure, A can be -S-; R¹ can be straight or branched C₁₋₄ alkylene; and X can be -OH.

In one embodiment of the present disclosure, A can be -SO₂-; R¹ can be straight or branched C₁₋₁₀ alkylene; R⁴ can be straight or branched C₃₋₁₀ alkenyl. In another embodiment of the present disclosure, A can be -SO₂-; R¹ can be straight or branched C₁₋₆ alkylene; X can be -OH or -OCOR⁴; and R⁴ can be straight or branched C₃₋₆ alkenyl. In another embodiment of the present disclosure, A can be -SO₂-; R¹ can be straight or branched C₁₋₄ alkylene; X can be -OH or -OCOR⁴; and R⁴ can be straight or branched C₃₋₄ alkenyl.

In one embodiment of the present disclosure, R² can be -R³Y, wherein R³ can be straight or branched C₁₋₁₀ alkylene; Y can be -OH, or -OCOR⁴, wherein R⁴ can be straight or branched C₃₋₁₀ alkenyl. In another embodiment of the present disclosure, R² can be -R³Y; wherein R³ can be straight or branched C₁₋₆ alkylene; and R⁴ can be straight or branched C₃₋₆ alkenyl. In another embodiment of the present disclosure, R² can be -R³Y; wherein R³ can be straight or branched C₁₋₃ alkylene; Y can be -OH or -OCOR⁴; and R⁴ can be straight or branched C₃₋₄ alkenyl.

The compound provided by the present disclosure can be any one of the following formulas (1-1) to (1-3):

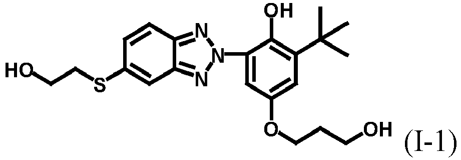

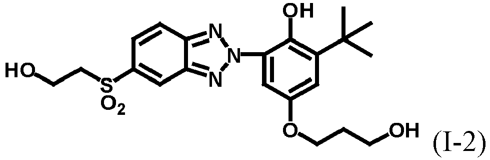

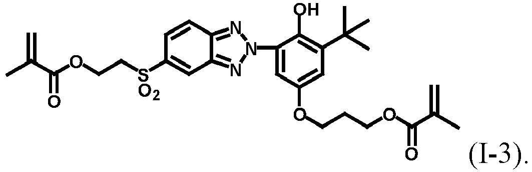

In the present disclosure, the term "alkyl(ene)" refers to straight and branched alkyl(ene), and includes, for example, straight or branched C₁₋₁₀ alkyl(ene), C₁₋₆ alkyl(ene) or C₁₋₄ alkyl(ene). Specific examples of alkyl(ene) include, but are not limited to, methyl(ene), ethyl(ene), n-propyl(ene), iso-propyl(ene), n-butyl(ene), sec-butyl(ene), iso-butyl(ene), tert-butyl(ene), pentyl(ene), neo-pentyl(ene) or hexyl(ene).

In the present disclosure, the term "alkenyl" includes straight or branched hydrocarbon groups with at least one double bond, and includes, for example, straight or branched C₃₋₁₀ hydrocarbon groups with at least one double bond, straight or branched C₃₋₆ hydrocarbon groups with at least one double bond, or straight or branch C₃₋₄ hydrocarbon groups with at least one double bond. Examples of the alkenyl include, but are not limited to propenyl or butenyl.

In the present disclosure, the term "cycloalkenyl(ene)" includes cyclic unsaturated hydrocarbon groups, which includes 3 to 10 carbon atoms (C₃₋₁₀), 5 to 8 carbon atoms (Cs-s) or 5 to 7 carbon atoms (C₅₋₇). Examples of the cycloalkenyl(ene) include, but are not limited to cyclopentenyl(ene), cyclohexenyl(ene) or cycloheptenyl(ene).

In the present disclosure, the term "aryl" includes 6-membered single aromatic ring, 10-membered double aromatic ring or 14-membered triple aromatic ring. Examples of the aryl include, but are not limited to phenyl, naphthyl, pyrenyl, anthryl or phenanthryl.

In the present disclosure, the term "aralkyl" refers to a moiety that the alkyl defined in the present disclosure coupled with at least one aryl.

In the present disclosure, the term "alkylene interrupted by an ester group" refers to a moiety that an ester group is introduced between two adjacent carbon atoms of the alkylene defined in the present disclosure, or an ester group is connected to one end of the alkylene defined in the present disclosure. In one embodiment of the present disclosure, "alkylene interrupted by an ester group" can be -C(=O)O-(alkylene).

Furthermore, the present disclosure also provides a composition with stability to photo-induced degradation, which comprises: (A) a photo-induced degradable organic material; and (B) the aforesaid novel compound of the present disclosure. Herein, a content of the novel compound of the present disclosure is 0.1% to 30% based on a weight of the photo-induced degradable organic material.

In one embodiment of the present disclosure, the aforesaid composition is used for forming a coating layer. In particular, the coating layer is formed on a substrate which is sensitive to electromagnetic radiation with a wavelength greater than 380 nm. The material of the substrate is not particularly limited, and can be glass, plastic, polymer, silicone hydrogel, resin, carbon fiber complex material, or a combination thereof.

Furthermore, the present disclosure also provides a glasses lens with anti-blue light effect, which comprises the aforesaid novel compound of the present disclosure. Herein, the novel compound of the present disclosure is applied to a substrate for the glasses lens to form an anti-UV or anti-blue light coating thereon.

In addition, the present disclosure further provides a protection film with anti-blue light effect, which comprises the aforesaid novel compound of the present disclosure. Herein, the novel compound of the present disclosure is applied to a substrate for the protection film to form an anti-UV or anti-blue light coating thereon. An example of the protection film can be a screen protector, but the present disclosure is not limited thereto.

Other novel features of the disclosure will become more apparent from the following detailed description when taken in conjunction with the accompanying drawings.

### BRIEF DESCRIPTION OF THE DRAWINGS

FIG. 1 is absorption spectra of compounds of Examples 1-2 and Comparative examples 1-2 (Comp exs. 1-2).
FIG. 2 is absorption spectra of compounds of Examples 2, 3 and 8.
FIG. 3 shows transmission results of compounds of Example 2 and Comparative example 1 (Comp ex. 1).

### DETAILED DESCRIPTION OF EMBODIMENT

The following embodiments when read with the accompanying drawings are made to clearly exhibit the above-mentioned and other technical contents, features and/or effects of the present disclosure. Through the exposition by means of the specific embodiments, people would further understand the technical means and effects the present disclosure adopts to achieve the above-indicated objectives. Moreover, as the contents disclosed herein should be readily understood and can be implemented by a person skilled in the art, all equivalent changes or modifications which do not depart from the concept of the present disclosure should be encompassed by the appended claims.

Unless specified otherwise, term "or" used in the present specification and claims includes meaning of "and/or".

The present disclosure is explained by the following embodiments, which are not used to limit the scope of the present disclosure. Unless specified otherwise, "%" used herein for indicating the amount of the contents or the objects in the following embodiments are weight percentage.

### Preparation example 1: Synthesis of the raw compound la

### Preparation of Compound la'

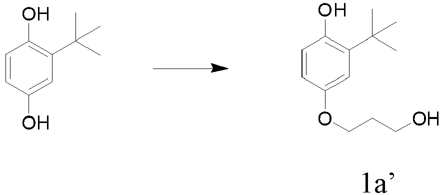

Tertiary butylhydroquinone (856.8 g) was dissolved in methanol (2.5 L), followed by adding NaOH (206.2 g), water (1L), chloropropanol (482.3 g) and KI (6.3 g). The mixture was heated at reflux temperature under N₂ automosphere for 48 hr. After the mixture was cooled down to room temperature, water (5.6 L) was added to dilute the mixture, and then the mixture was extracted with dichloromethane (4 L). The combined dichloromethane layer was washed with water, dried with anhydrous sodium sulfate and concentrated. The residue was purified with reduced pressure distillation, and the distillate was recrystallized with toluene for further purification to obtain white solids (396.0 g, m.p. 78.5-80.5°C).

### Preparation of Compound 1a

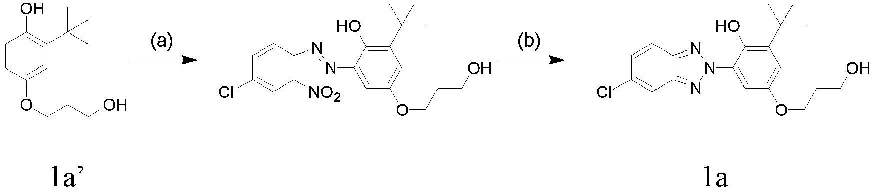

### Step (a)

4-Chloro-2-nitroaniline (90.6 g) and concentrated HCl (180 mL) was mixed and stirred at room temperature for 1 hr, and the mixture was diluted with water (160 mL) and ice (300 g). At -5°C, sodium nitrite (37.7 g) was dissolved in water (140 mL) and then added into the mixture. After adding sodium nitrite, the mixture was stirred at 0°C for 1 hr, and then sulfamic acid was addd therein unitl the result on the potassium iodide-starch test paper showed negative. Then, the mixture was filtered, the filtrate was added to a mixture containing the compound 1a' (112.2 g), NaOH (60 g) and water (2 L) at -5°C to 0°C under stirring. After adding about 1/3 diazonium solution, 10% NaOH aqueous solution (400 mL) was added together with the diazonium solution into the mixture, and the addition of the diazonium solution and the NaOH aqueous solution was finished at the same time. The addition of the diazonium solution and the NaOH aqueous solution was held at 0°C or less. After addition, the mixture was stirred for further 2 hr at the same temperature. Then, the mixture was placed to let the temperature of the mixture back to room temperature. The azo dye was separated by acidification with HCl, filered and washed with water. The obtained azo dye was directly used in the next step without further purification.

### Step (b)

The azo dye obtained in the step (a) was dissolved in ethanol (1.5 L), and a glucose solution (glucose (180 g) dissolved in 2 N NaOH aqueous solution (1.5 L)) was slowly added into the azo dye solution. The temperature was kept at 30°C or less, and the thin layer chromatography was used to trace the completion of the reaction. Then, fresh activated Zn powders (165 g) were added. The mixture was stirred at room temperature for 3 hr, diluted with water (1 L) and stirred for 15 min, and then left to stand for 1 hr. The precipitate was filtered and seperated, followed by washing with water. The filter cake was further extracted with hot ethanol (2 L) until only Zn powders were left in the filter cake. The extract was cooled down to room temperature, and the solid was filtered and separated, followed by washing with cold ethanol. After vacuum drying, yellow solids can be obtained (67.8 g, m.p. 134.7° C).

¹H NMR (400MHz, CDCl₃): δ 11.35 (1H), 7.92 (1H), 7.87 (1H), 7.80 (1H), 7.43 (1H), 7.02 (1H), 4.20 (2H), 3.91 (2H), 2.10 (2H), 1.80 (1H), 1.49 (9H)

TGA (5% weight loss): 246.2°C

### Example 1: Synthesis of Compound 2a (i.e. the compound (1-1) of the present disclosure)

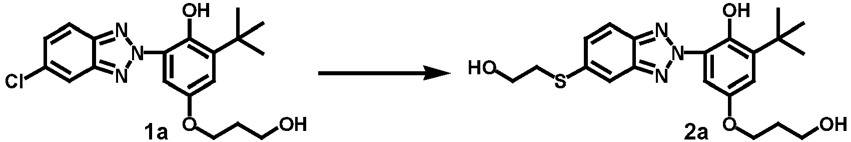

To a flask (20 mL), the compound **1a** (0.75 g), KOH (0.52 g), KI (0.09 g), 2-mercaptoethanol (0.5 mL), *N*-methylpyrrolidone (2 mL) were added, and the mixture was heated to 100°C and stirred for 12 hr. After the mixture was cooled down to room temperature, the mixture was acidified with IN HCl aqueous solution to pH 5. The mixture was extracted with toluene (100 mL), ethyl acetate (50 mL) and water (100 mL). The water layer was removed, and the organic layer was dried with MgSO₄ to remove water. After reduced pressure concentration and purification with column chromatography, the compound **2a** was obtained (0.21 g, 25.2%).

¹H NMR (CDCl₃, 400MHz): 1.49 (s, 9H), 1.86 (s, 1H), 2.10 (quint, *J* = 5.9 Hz, 2H), 3.25 (t, *J* = 5.8 Hz, 2H), 3.80-3.95 (m, 4H), 4.20 (t*, J* = 6.0 Hz, 2H), 7.06 (d, *J* = 3.0 Hz, 1H), 7.42 (dd, *J* = 1.4, 8.8 Hz, 1H), 7.79 (d, *J* = 3.0 Hz, 1H), 7.82 (d, *J* = 8.8 Hz, 1H), 7.83 (d, *J* = 1.4 Hz, 1H), 11.42 (s, 1H); ¹³C NMR (CDCl₃, 100MHz): 29.5, 32.2, 35.8, 36.9, 60.5, 60.8, 103.1, 115.8, 117.2, 118.1, 125.3, 129.9, 136.0, 141.1, 141.6, 143.3, 143.6, 151.2; HRMS(ESI⁺, M+H): calc. 418.1801, found 418.1804.

### Example 2: Synthesis of Compound 2b (i.e. the compound (1-2) of the present disclosure)

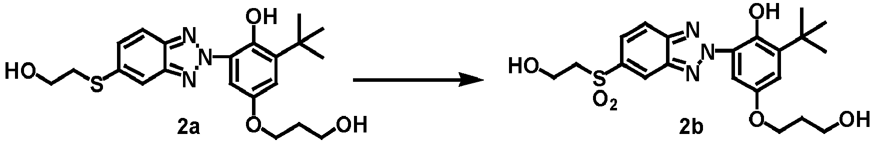

To a flask (250 mL), the compound **2a** (0.75 g), Na₂WO₄·2H₂O (0.1 g), 90% formic acid aqueous solution (9 mL), toluene (50 mL), 30% H₂O₂ aqueous solution (9 mL) were added, and the mixture was heated to 70°C and stirred for 6 hr. After the mixture was cooled down to room temperature, the mixture was extracted with toluene (100 mL) and water (10 mL). The water layer was further extracted with toluene (100 mL) and combined with the organic layer. The organic layer was washed with saturated brine (100 mL) and dried with MgSO₄, followed by reduced pressure concentration and purification with chromatography to obtain the compound **2b** (0.23 g, 28.3%).

¹H NMR(CDCl₃, 400MHz): 1.49 (s, 9H), 2.04-2.17 (m, 2H), 2.80 (s, 1H), 3.47 (dt, *J* = 6.0, 2.4 Hz, 2H), 3.92 (t*, J* = 6.0 Hz, 2H), 4.09 (t, *J* = 5.2 Hz, 2H), 4.21 (t, *J* = 6.0 Hz, 2H), 7.07 (d, *J* = 3.2Hz, 1H), 7.83 (d, *J* = 3.2Hz, 1H), 7.94 (d, *J* = 9.1 Hz, 1H), 8.12 (dd, *J* = 0.8, 9.1 Hz, 1H), 8.66 (d, *J* = 0.8 Hz, 1H), 11.22 (s, 1H); ¹³C NMR(CDCl₃, 100MHz): 29.5, 32.2, 35.9, 56.5, 58.6, 60.5, 66.5, 103.2, 118.6, 119.5, 120.8, 125.0, 138.3, 141.5, 141.7, 144.1, 144.4, 151.4; HRMS(ESI⁺, M+H): calc. 450.1699, found 450.1710.

### Example 3: Synthesis of Compound 2c (i.e. the compound (1-3) of the present disclosure)

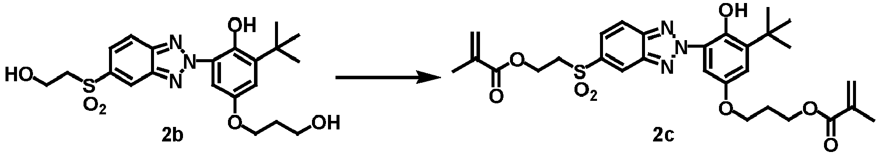

To a flask (250 mL), the compound **2b** (0.85 g), hydroquinone (0.1 g), *p*-toluenesulfonic acid (0.1 g), methacrylic acid (0.5 mL) and toluene (125 mL) were added, and the mixture was heated and stirred for 16 hr under reflux. After the mixture was cooled down to room temperature, the mixture was extracted with saturated NaHCO₃ aqueous solution (100 mL). The water layer was further extracted with toluene (50 mL) and combined with the organic layer. The organic layer was washed with saturated brine (100 mL) and dried with MgSO₄. After reduced pressure concentration and purification with chromatography, the product was recrystallized with methanol/toluene to obtain the compound **2c** (0.34 g, 30.7%).

¹H NMR(CDCl₃, 400MHz): 1.50 (s, 9H), 1.71 (d, *J* = 1.4 Hz, 3H), 1.97 (d, *J* = 1.4 Hz, 3H), 2.23 ( quint, *J* = 6.2 Hz, 2H), 3.63 (t, *J* = 6.0 Hz, 2H), 4.17 (t, *J* = 6.0 Hz, 2H), 4.40 (t, *J* = 6.2 Hz, 2H), 4.56 (t, *J* = 5.8 Hz, 2H), 5.37 (dd, *J* = 0.9, 1.4 Hz, 1H), 5.58 (dd, *J* = 0.9, 1.4 Hz, 1H), 5.75 (s, 1H), 6.14 (s, 1 H), 7.08 (d, *J* = 2.8 Hz, 1H), 7.81 (d, *J* = 2.8 Hz, 1H), 7.93 (dd, *J* = 1.2 Hz, 9.2 Hz, 1H), 8.11 (d, *J* = 9.2 Hz, 1H), 8.66 (d, *J* = 1.2 Hz, 1H), 11.24 (s, 1H); ¹³C NMR(CDCl₃, 100MHz): 18.1, 18.5, 28.8, 29.5, 35.9, 55.4, 58.0, 61.7, 65.3, 103.0, 119.0, 119.4, 121.0, 124.9, 125.1, 125.7, 126.6, 135.3, 136.5, 138.7, 141.6, 144.1, 144.4, 151.5, 166.6, 167.5; HRMS(ESI⁺, M+H): calc. 586.2223, found 586.2227.

### Example 4: Synthesis of Compound 2d (i.e. the compound (1-4) which is not according to the invention)

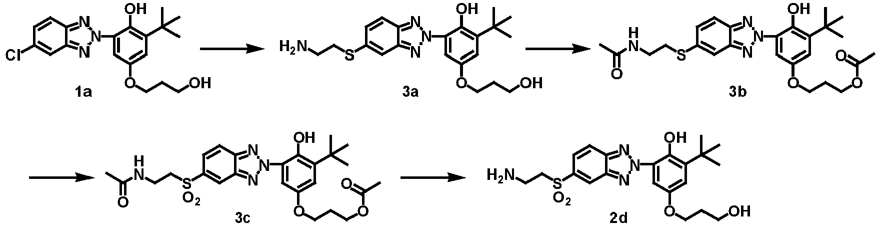

To a flask (25 mL), the compound **1a** (0.3 g), K₂CO₃ (2.0 g), 2-aminoethanethiol (1.2 g) and *N*-methylpyrrolidone (2 mL) were added, the mixture was heated to 100°C and stirred for 12 hr. After cooling down to room temperature, the mixture was added into water (100 mL), and IN HCl aqueous solution was added therein until the pH value of the mixture was 5. Isopropanol (25 mL), toluene (75 mL) and NaCl (10 g) was added into the mixture, followed by extraction. The water layer was extracted with isopropanol (25 mL) and toluene (75 mL) and combined with the organic layer. The organic layer was washed with saturated brine (100 mL), followed by reduced pressure concentration and purification with chromatography to obtain an intermediate **3a,** which was all used in the sequential reaction.

¹H NMR(Pyridine-*d*⁵, 400MHz): 1.59 (s, 9H), 2.29 (quint, *J* = 6.2 Hz, 2H), 3.82 (t, *J* = 7.1 Hz, 2H), 4.05 (t, *J* = 7.1 Hz, 2H), 4.13 (t, *J* = 6.2 Hz, 2H), 4.37 (t*, J* = 6.2 Hz, 2H), 7.24 (d*, J* = 2.6Hz, 1H), 7.55 (d*, J* = 9.0 Hz, 1H), 7.90 (d, *J* = 9.0 Hz, 1H), 8.00 (d, *J* = 2.6Hz, 1H), 8.19 (s, 1 H); HRMS(ESI⁺, M+H): calc. 417.1960, found 417.1971.

The intermediate **3a** was mixed with ethyl acetate (30 mL), *tert*-butanol (30 mL), acetic anhydride (3 mL) and triethylamine (3 mL), and the mixture was heated to 70°C and stirred. After the intermediate **3a** was dissolved completely, the mixture was cooled down to room temperature. The mixture was extracted with water (100 mL) and ethyl acetate (100 mL), and the water layer was further extracted with ethyl acetate (50 mL) and combined with the organic layer. The organic layer was washed with saturated brine (50 mL), followed by reduced pressure concentration to obtain an intermediate **3b,** which was all used in the sequential reaction.

The intermediate **3b** was mixed with Na₂WO₄·2H₂O (0.1 g), 30% H₂O₂ aqueous solution (15 mL), water (5 mL) and isopropanol (50 mL), and the mixture was stirred at room temperature for 16 hr. The mixture was extracted with water (100 mL) and ethyl acetate (100 mL), and the water layer was further extracted with ethyl acetate (50 mL) and combined with the organic layer. The organic layer was washed with saturated brine (100 mL), followed by reduced pressure concentration to obtain an intermediate **3c,** which was all used in the sequential reaction. HRMS(ESI+, M+H): calc. 533.2070, found 533.2074.

The intermediate **3c** was mixed with 85% phosphoric acid (3 mL) and water (1 mL), and the mixture was heated and stirred for 8 hr under reflux. After the mixture was cooled down to room temperature, the mixture was added into water (100 mL), and neutralized with 45% NaOH aqueous solution to pH 6.5. Isopropanol (100 mL), toluene (100 mL) and NaCl (10 g) was added into the mixture, followed by extraction. The water layer was further extracted with isopropanol (50 mL) and toluene (50 mL) and combined with the organic layer. The organic layer was washed with saturated brine (100 mL), followed by reduced pressure concentration and crystallized with toluene/methanol to obtain the compound **2d** (0.12 g). The final yield after the aforesaid four steps was 33.5%.

¹H NMR(DMSO-*d*⁶, 400MHz): 1.45 (s, 9H), 1.89 ( quint, *J* = 6.3 Hz, 2H), 2.94 (s, 2H), 3.59 (t, *J* = 6.3 Hz, 2H), 3.65 (s, 2H), 4.06 (t, *J* = 6.3 Hz, 2H), 7.00 (d, *J* = 2.8 Hz, 1H), 7.49 (t, *J* = 2.8 Hz, 1H), 7.98 (d, *J* = 8.8 Hz, 1H), 8.35 (d, *J* = 8.8 Hz, 1H), 8.72 (d, *J* = 2.8 Hz, 1H); ¹³C NMR(DMSO-*d*⁶, 100MHz): 29.2, 32.1, 34.7, 35.3, 55.3, 57.3, 65.3, 104.9, 117.3, 119.9, 120.5, 124.8, 126.4, 137.9, 140.6, 141.9, 143.0, 144.6, 151.1; HRMS(ESI⁺, M+H): calc. 449.1859, found 449.1859.

### Preparation example 2: Synthesis of the raw compound 1b

### Synthesis of the precursor compound 4

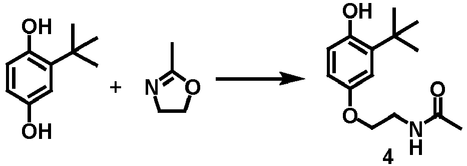

To a flask (250 mL), *tert*-butylhydroquinone (33.24 g) and 2-methyl-2-oxazoline (20.42 g) were added, and the mixture was heated to 160°C and stirred for 16 hr. After the mixture was cooled down to room temperature, the mixture was extracted with toluene (100 mL), ethyl acetate (200 mL) and water (100 mL). The water layer was removed, and the organic layer was further washed with saturated brine (100 mL). The organic layer was dried and concentrated. After reduced pressure distillation at 0.25 mbar, and the distillate obtained in the region with the boiling point between 215°C and 220°C contained the compound **4** (30.31 g, 42.2%).

¹H NMR (Acetone-*d*⁶, 400MHz): 1.38 (s, 9H), 1.93 (s, 3H), 3.53 (q, *J* = 5.6 Hz, 2H), 3.94 (t, *J* = 5.6 Hz, 2H), 6.58 (dd, *J* = 2.8, 8.8 Hz, 1H), 6.74 (d, *J* = 8.8 Hz, 1H), 6.78 *(d, J* = 2.8 Hz, 1H), 7.51 (br s, 1H), 8.18 (br s, 1H); ¹³C NMR (Acetone-*d*⁶, 100MHz): 22.8, 29.8, 35.2, 39.8, 67.8, 112.2, 115.0, 117.2, 137.6, 150.8, 152.7, 170.8.

### Synthesis of the raw compound 1b

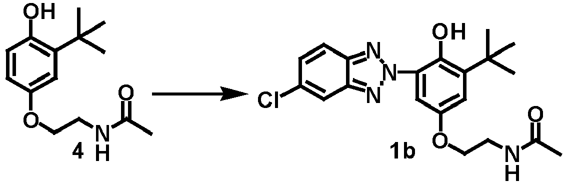

4-Chloro-2-nitroaniline (16 g), a dispersant (0.05 g), concentrated HCl (50 ml) and ice (100 g) were mixed and stirred for 1 hr. After the mixture was cooled down to 5°C, a sodium nitrite aqueous solution (6.8 g sodium nitride dissolved in 50 mL water) was slowly added. After adding the sodium nitrite aqueous solution, the mixture was stirred for further 0.5 hr, followed by adding ammonium sulfamate (0.3 g) and stirring for 0.5 hr. After adding diatomaceous earth (0.3 g) and stirring for 0.5 hr, the mixture was filtered to obtain an azo solution, and the azo solution was placed at low temperature (<5°C) for the sequential reaction.

The compound **4** (18.71 g), a dispersant (0.1 g), 45% NaOH aqueous solution (10 g), ice (100 g) and ethanol (500 mL) were mixed and stirred for 1 hr. After cooling down to 5°C, the prepared azo solution was slowly added into the mixture dropwise, and 45% NaOH aqueous solution was added when needed to control the pH value between 8 and 10.5. After adding the azo solution, the temperature of the mixture was back to room temperature, and the mixture was stirred for 16 hr. Then, 45% NaOH aqueous solution (66 g) as added, and the mixture was heated and stirred for 0.5 hr under reflux. Sodium dithionite (16 g) was added and stirred for 1 hr, and then sodium dithionite (16 g) was further added and stirred for 1 hr, and then sodium dithionite (32 g) and 45% NaOH aqueous solution (16 g) was further added and stirred for further 1 hr. After the mixture was back to room temperature, the mixture was filtered and the solids was collected to obtain the compound **1b** (13.3 g, 44.3%).

¹H NMR (DMSO-*d*⁶, 400MHz): 1.45 (s, 9H), 1.84 (s, 3H), 3.43 (q, *J* = 5.2 Hz, 2H), 4.02 (t, *J* = 5.2 Hz, 2H), 7.00 (s, 1H), 7.53 (s, 1H), 7.59 (d, *J* = 9.0 Hz, 1H), 8.12 (s, 1 H), 8.14 (d, *J* = 9.0 Hz, 1H), 8.25 (s, 1H), 10.64 (br s, 1H); ¹³C NMR (DMSO-*d*⁶, 100MHz): 22.5, 29.2, 35.3, 38.3, 67.1, 104.6, 116.9, 119.7, 126.0, 129.0, 132.4, 140.4, 141.4, 142.8, 143.2, 150.7, 169.5.

### Example 5: Synthesis of Compound 2e (i.e. the compound (1-5) which is not according to the invention)

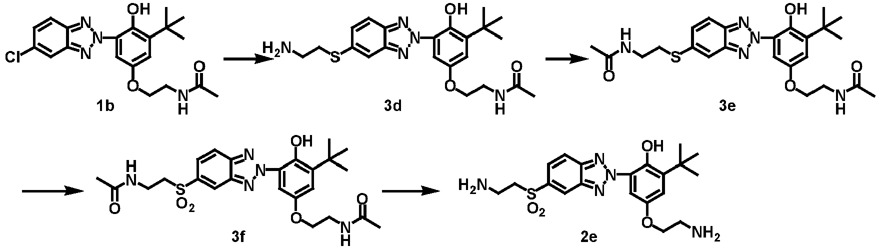

To a flask (25 mL), the compound **1b** (1.25 g), potassium carbonate (4.0 g), 2-aminoethanethiol (1.2 g) and *N*-methyl-2-pyrrolidone (10 mL) were added, and the mixture was heated to 100°C and stirred for 12 hr. After cooling down to room temperature, the mixture was added into water (300 mL) and acidified with 1 N HCl aqueous solution to pH 6. The mixture was extracted with isopropanol (25 mL), toluene (75 mL) and NaCl (20 g). The water layer was extracted with isopropanol (25 mL) and toluene (75 mL) and then combined with the organic layer. The organic layer was washed with saturated brine (100 mL), concentrated under reduced pressure, and purified with column chromatography to obtain an intermediate **3d,** which was all used in the sequential reaction.

The intermediate **3d** was mixed with ethyl acetate (50 mL), *tert*-butanol (9 mL), acetic anhydride (12 mL) and triethylamine (9 mL), the mixture was heated to 70°C and stirred. After the intermediate **3d** was completely dissolved, the mixture was cooled down to room temperature. The mixture was extracted with water (100 mL) and ethyl acetate (100 mL). The water layer was further extracted with ethyl acetate (50 mL) and then combined with the organic layer. The organic layer was washed with saturated brine (50 mL) and concentrated under reduced pressure to obtain an intermediate **3e,** which was all used in the sequential reaction.

The intermediated **3e** was mixed with Na₂WO₄·2H₂O (0.1 g), 30% H₂O₂ aqueous solution (15 mL), water (5 mL) and isopropanol (50 mL), and the mixture was stirred at room temperature for 16 hr. The mixture was extracted with water (100 mL) and ethyl acetate (100 mL). The water layer was further extracted with ethyl acetate (50 mL) and then combined with the organic layer. The organic layer was washed with saturated brine (100 mL) and concentrated under reduced pressure to obtain an intermediate **3f,** which was all used in the sequential reaction.

The intermediated **3f** was mixed with 85% phosphoric acid (12 mL) and water (3 mL), and the mixture was heated under reflux for 8 hr. After cooling down to room temperature, the mixture was added into water (200 mL), and neutralized with 45% NaOH aqueous solution to pH 6.5. The mixture was filtered to collect solids, and the solids were washed with water (50 mL). The filter cake was recrystallized with isopropanol to obtain the compound **2e** (0.13 g). The total yield after four steps was 9.7%.

¹H NMR(DMSO-*d*⁶, 400MHz): 1.46 (s, 9H), 2.92 (t, *J* = 7.2 Hz, 2H), 3.18 (t, *J* = 4.8 Hz, 2H), 3.64 (t, *J* = 7.2 Hz, 2H), 4.22 (t, J = 4.8Hz, 2H), 7.11 (d, *J* = 2.8 Hz, 1H), 7.55 (d, *J* = 2.8 Hz, 1H), 7.98 (d, *J* = 2.8 Hz, 1H), 8.00 (dd, *J* = 1.2, 9.2 Hz, 1H), 8.38 (d, *J* = 9.2 Hz, 1H), 8.74 (d, *J* = 1.2 Hz, 1H); ¹³C NMR(DMSO-*d*⁶, 100MHz): 29.2, 35.2, 35.3, 38.6, 56.0, 65.7, 105.8, 117.7, 119.9, 120.5, 124.8, 126.5, 138.1, 140.7, 142.0, 143.6, 144.7, 150.3; HRMS(ESI⁺, M+H): calc. 434.1862, found 434.1863.

### Example 6: Synthesis of Compound 2f (i.e. the compound (1-6) which is not according to the invention)

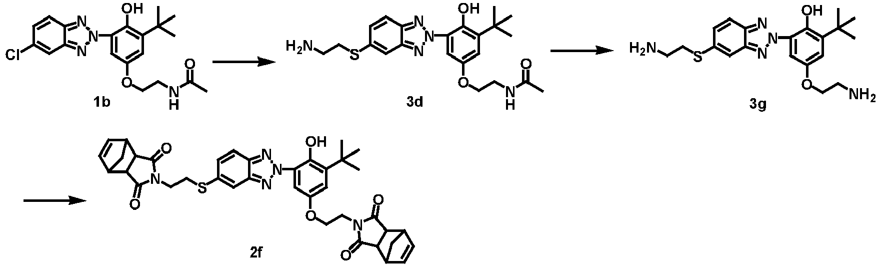

To a flask (25 mL), the compound **1b** (2.88 g), potassium carbonate (3.0 g), 2-aminoethanethiol (3.0 g) and *N*-methyl-2-pyrrolidone (10 mL) were added, the mixture was heated to 100°C and stirred for 12 hr. After cooling down to room temperature, the mixture was added into water (300 mL) and acidified with 1 N HCl aqueous solution to pH 5. The mixture was filtered, and the collected solids were washed with water (100 mL) to obtain an intermediate **3d** (1.5 g), which was all used in the sequential reaction.

The intermediate **3d** was mixed with 85% phosphoric acid (12 mL) and water (3 mL), and the mixture was stirred and heated under reflux for 8 hr. After cooling down to room temperature, the mixture was added into water (200 mL), and neutralized with 45% NaOH aqueous solution to pH 6.5. The mixture was filtered, and the collected solids were washed with water (50 mL). The filter cake was recrystallized with isopropanol to obtain an intermediate **3g** (5 g).

The intermediate **3g** (0.15 g) was added into 5-norbornene-2,3-dicarboxylic anhydride (0.15 g) and toluene (20 mL), and the mixture was stirred under reflux for 16 hr. After cooling down to room temperature, the mixture was concentrated under reduced pressure and purified with column chromatography to obtain the compound **2f** (0.1 g). The total yield of the three steps was 15.1%.

¹H NMR(CDCl₃, 400MHz): 1.47 (dt, *J* = 8.8, 1.6 Hz, 1H), 1.48 (s, 9H), 1.54 (dt, *J* = 8.8, 1.6 Hz, 1H), 1.73 (dt, *J* = 8.8, 1.6 Hz, 1H), 1.74 (dt, *J* = 8.8, 1.6 Hz, 1H), 3.10 (t*, J* = 7.6 Hz, 2H), 3.24-3.27 (m, 1H), 3.29-3.32 (m, 1H), 3.38-3.43 (m, 4H), 3.66 (t*, J* = 7.6 Hz, 2H), 3.81 (t*, J* = 5.6 Hz, 2H), 4.10 (t, *J* = 5.6 Hz, 2H), 6.08 (t, *J* = 1.6 Hz, 2H), 6.14 (t, *J* = 1.6 Hz, 2H), 6.95 (d, *J* = 3.0 Hz, 1H), 7.40 (dd, *J* = 8.8, 3.2 Hz, 1H), 7.72 (d, *J* = 3.0 Hz, 1H), 7.83 (d, *J* = 8.8 Hz, 1H), 7.90 (d, *J* = 3.2 Hz, 1H), 11.46 (s, 1H); ¹³C NMR(CDCl₃, 100MHz): 29.5, 30.3, 35.7, 37.3, 37.8, 45.4, 45.9, 52.2, 52.3, 64.9, 103.2, 115.3, 117.0, 118.0, 125.2, 129.5, 134.4, 134.6, 135.7, 140.9, 141.5, 143.3, 143.6, 150.7, 177.5, 177.6; HRMS(ESI⁺, M+H): calc. 694.2699, found 694.2775.

### Preparation example 3: Synthesis of the raw compound 1c

### Synthesis of the raw compound 1c'

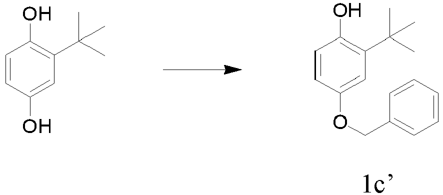

The preparation process can be referred to that for the compound **1a'.** Herein, the starting materials were *tert*-butylhydroquinone (856.8 g) and benzyl bromide (960.0 g), and light yellow liquid (670.0 g) was obtained.

### Synthesis of the raw compound 1c

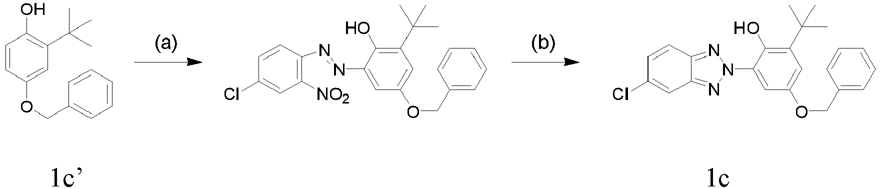

The preparation process can be referred to that for the compound **1a.** Herein, the starting material was the raw compound **1c'** (450.0 g), and yellow solids (138.0 g, m.p. 130.7°C) were obtained.

¹H NMR (400MHz, CDCl₃): δ 11.36 (1H), 7.80-8.00 (3H), 7.49 (2H), 7.30-7.45 (4H), 7.10 (1H), 5.11 (2H), 1.48 (9H); HRMS ESI [M-H]⁻: calc. 406.1322, found 406.1324.

### Example 7: Synthesis of Compound 2g (i.e. the compound (1-7) which is not according to the invention)

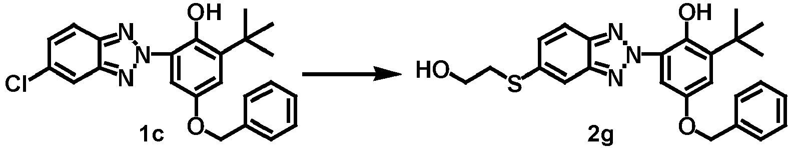

To a flask (20 mL), the compound **1c** (2.0 g), KOH (0.6 g), potassium carbonate (10.0 g), 2-mercaptoethanol (3 mL) and *N*-methylpyrrolidone (20 mL) were added. The mixture was heated to 140°C and stirred for 8 hr. After cooling down to room temperature, the mixture was acidified with 1 N HCl aqueous solution to pH 5. The mixture was extracted with toluene (100 mL), ethyl acetate (50 mL) and water (100 mL). The water layer was removed, and the organic layer was dried with MgSO₄ to remove water. After reduced pressure concentration and purification with column chromatography, the compound **2g** (0.8 g, 36.3%) was obtained.

¹H NMR (CDCl₃, 400MHz): 1.48 (s, 9H), 3.25 (t, *J* = 6.0 Hz, 2H), 3.87 (t, *J* = 6.0 Hz, 2H), 5.12 (s, 2H), 7.09 (d, *J* = 3.2 Hz, 1H), 7.29-7.55 (m, 6 H), 7.77-7.93 (m, 3H), 11.44 (s, 1 H); ¹³C NMR (CDCl₃, 100MHz): 29.6, 35.8, 36.9, 60.5, 70.9, 103.4, 115.9, 117.6, 118.1, 125.3, 127.9, 128.2, 128.8, 129.9, 136.0, 137.0, 141.1, 141.6, 143.3, 143.7, 151.3; HRMS(ESI⁺, M+H): calc. 450.1851, found 450.1863.

### Example 8: Synthesis of Compound 2h (i.e. the compound (1-8) which is not according to the invention)

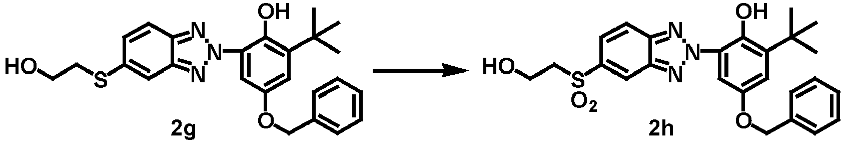

To a flask (250 mL), the compound **2g** (0.4 g), Na₂WO₄·2H₂O (0.1 g), 30% H₂O₂ aqueous solution (15 mL), isopropanol (50 mL) and water (5 mL) were added and stirred for 16 hr. The mixture was extracted with toluene (100 mL) and NaCl (10 g). The water layer was further extracted with toluene (100 mL) and combined with the organic layer. The organic layer was washed with saturated brine (100 mL), dried with MgSO₄, concentrated under reduced pressure and purified with column chromatography to obtain the compound **2h** (0.28 g, 65.3%).

¹H NMR(CDCl₃, 400MHz): 1.49 (s, 9H), 2.66 (*t*, J = 2.4 Hz, 1H), 3.47 (dt, *J* = 2.4, 5.2 Hz, 2H), 4.09 (t, *J* = 5.2 Hz, 2H), 5.14 (s, 2H), 7.16 (d, *J* =2.8 Hz, 1H), 7.30-7.47 (m, 3H), 7.50 (d, *J* = 7.2Hz, 2H), 7.89-7.99 (m, 2H), 8.14 (d, *J* = 9.2 Hz, 1H), 8.68 (s, 1H), 11.25 (s, 1H); ¹³C NMR(CDCl₃, 100MHz): 29.5, 35.9, 56.6, 58.6, 71.0, 103.5, 119.0, 119.6, 120.9, 125.0, 127.9, 128.3, 128.8, 136.8, 138.3, 141.6, 141.7, 144.3, 144.5, 151.4; HRMS(ESI⁺, M+H): calc. 482.1750, found 482.1764.

### Example 9: Synthesis of Compound 2i (i.e. the compound (1-9) which is not according to the invention)

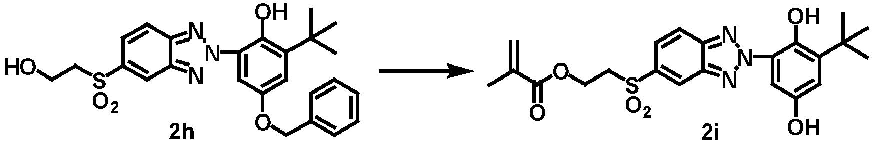

To a flask (250 mL), the compound **2h** (0.2 g), hydroquinone (0.1 g), *p*-toluenesulfonic acid (0.1 g), methacrylic acid (0.5 mL) and toluene (75 mL) were added, and the mixture was heated and stirred under reflux for 16 hr. After cooling down to room temperature, the mixture was extracted with toluene (25 mL) and saturated NaHCO₃ aqueous solution (100 mL). The water layer was further extracted with toluene (50 mL) and combined with the organic layer. The organic layer was washed with saturated brine (100 mL), dried with MgSO₄, concentrated under reduced pressure, purified with column chromatography and recrystallized with methanol/toluene to obtain the compound **2i** (0.15 g, 78.6%).

¹H NMR(CDCl₃, 400MHz): 1.49 (s, 9H), 1.72 (d, *J* = 1.6 Hz, 3H), 1.97 (d, *J* = 1.2 Hz, 3H), 3.64 (t, *J* = 6.0 Hz, 2H), 4.57 (t, *J* = 6.0 Hz, 2H), 5.38 (d, *J* = 1.6 Hz, 1H), 5.76 (s, 1H), 7.02 (d, *J* = 2.8 Hz, 1H), 7.78 (d, *J* = 2.8 Hz, 1H), 7.92 (dd, *J* = 1.2 Hz, 9.2 Hz, 1H), 8.08 (d, *J* = 9.2 Hz, 1H), 8.64 (d, *J =* 1.2 Hz, 1H), 11.13 (s, 1H); ¹³C NMR(CDCl₃, 100MHz): 18.1, 29.5, 35.9, 55.4, 58.0, 105.6, 117.9, 119.5, 121.1, 125.1, 126.8, 135.3, 138.6, 141.6, 141.7, 143.8, 144.4, 148.1, 166.7; HRMS(ESI⁺, M+H): calc. 460.1542, found 460.1549.

### Preparation example 4: Synthesis of the raw compound 1d

### Synthesis of the raw compound 1d'

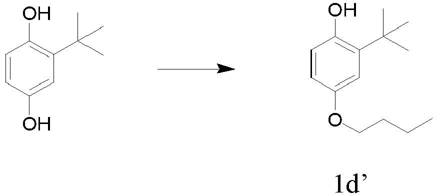

The preparation process can be referred to that for the compound **1a'.** Herein, the starting materials were *tert*-butylhydroquinone (856.8 g) and butyl bromide (706.3 g), and light yellow liquid (590.1 g) was obtained.

### Synthesis of the raw compound 1d

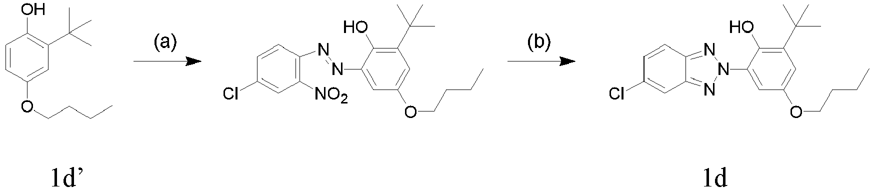

The preparation process can be referred to that for the compound **1a.** Herein, the starting material was the raw compound **1d'** (450.0 g), and yellow solids (159.6 g, m.p. 101.6°C) were obtained.

HRMS ESI [M-H]⁻: calc. 372.1479, found 372.1477

¹H NMR (400MHz, CDCl₃): δ 11.31 (1H), 7.91 (1H), 7.85 (1H), 7.75 (1H), 7.41 (1H), 7.02 (1H), 4.03 (2H), 1.81 (2H), 1.54 (2H), 1.49 (9H), 1.00 (3H)

### Example 10: Synthesis of Compound 2j (i.e. the compound (1-10) which is not according to the invention)

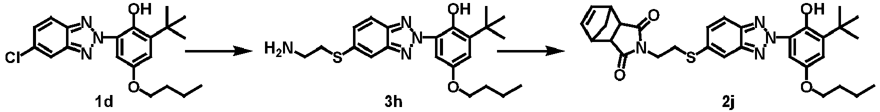

To a flask (25 mL), the compound **1d** (1.0 g), potassium carbonate (1.6 g), 2-aminoethanethiol (1.0 g), and *N*-methylpyrrolidone (10 mL) were added. The mixture was heated to 120°C and stirred for 16 hr. After cooling down to room temperature, the mixture was added into water (300 mL) and acidified with IN HCl aqueous solution to pH 5. The mixture was extracted with toluene (200 mL) and NaCl (10 g). The water layer was extracted with toluene (100 mL) and combined with the organic layer. The organic layer was washed with saturated brine (100 mL), concentrated under reduced pressure and purified with column chromatography to obtain an intermediate **3h** (0.3 g), which was all used in the sequential reaction. HRMS(ESI⁺, M+H): calc. 415.2168, found 415.2164.

The intermediate **3h** was mixed with 5-norbornene-2,3-dicarboxylic anhydride (1.0 g), dimethylformamide (20 mL) and toluene (20 mL), and the mixture was stirred under reflux for 16 hr. After cooling down to room temperature, the mixture was extracted with toluene (200 mL), NaCl (10 g) and saturated ammonium chloride aqueous solution (100 mL). The water layer was further extracted with ethyl acetate (100 mL) and combined with the organic layer. The organic layer was washed with saturated brine (100 mL), concentrated under reduced pressure and purified with column chromatography to obtain the compound **2j** (0.21 g). The total yield of the two steps was 14.0%.

¹H NMR(CDCl₃, 400MHz): 1.03 (t, *J* = 7.6 Hz, 3H), 1.45-1.65 (m, 12H), 1.70-1.90 (m, 3H), 3.08 (t, *J* = 7.2 Hz, 2H), 3.20-3.30 (m, 2H), 3.40-3.47 (m, 2H), 3.65 (t*, J* = 7.2 Hz, 2H), 4.04 (t*, J* = 6.4 Hz, 2H), 6.14 (s, 2H), 7.01 (d, *J* = 2.8 Hz, 1H), 7.39 (dd, *J* = 9.2, 1.4 Hz, 1H), 7.76 (d, *J* = 2.8 Hz, 1H), 7.83 (d, *J* = 9.2 Hz, 1H), 7.90 (d, *J* = 1.4 Hz, 1H), 11.43 (s, 1H); ¹³C NMR(CDCl₃, 100MHz): 14.0, 19.4, 29.5, 30.3, 31.6, 35.7, 37.4, 45.1, 45.9, 52.3, 68.5, 102.8, 115.4, 117.2, 118.0, 125.2, 129.5, 134.6, 135.6, 140.8, 141.5, 143.3, 143.4, 151.5, 177.5; HRMS(ESI⁺, M+H): calc. 561.2536, found 561.2608.

### Example 11: Synthesis of Compound 2k (i.e. the compound (1-11) which is not according to the invention)

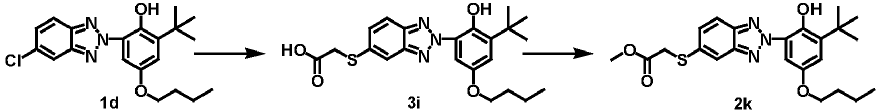

To a flask (25 mL), the compound **1d** (1.0 g), potassium carbonate (1.6 g), mercaptoacetic acid (1.0 mL) and *N*-methylpyrrolidone (10 mL) were added, and the mixture was heated to 140°C and stirred for 16 hr. Then, potassium carbonate (1.6 g) was further added therein and the mixture was further heated for 16 hr. After cooling down to room temperature, the mixture was added into water (300 mL) and acidified with IN HCl aqueous solution to pH 5. The mixture was extracted with isopropanol (25 mL), ethyl acetate (75 mL) and NaCl (10 g). The water layer was extracted with isopropanol (25 mL) and ethyl acetate (75 mL) and combined with the organic layer. The organic layer was washed with saturated brine (100 mL) and concentrated under reduced pressure to obtain an intermediate **3i,** which was all used in the sequential reaction.

The intermediate **3i** was mixed with *p*-toluenesulfonic acid (0.5 g), toluene (20 mL) and methanol (100 mL), and the mixture was stirred under reflux for 16 hr. After cooling down to room temperature, the mixture was extracted with toluene (200 mL), NaCl (10 g) and saturated NaHCO₃ aqueous solution (100 mL). The water layer was further extracted with ethyl acetate (100 mL) and combined with the organic layer. The organic layer was washed with saturated brine (100 mL), concentrated under reduced pressure and purified with column chromatography to obtain the compound **2k** (0.34 g). The total yield of the two steps was 28.7%.

¹H NMR(CDCl₃, 400MHz): 1.01 (t*, J* = 7.2 Hz, 3H), 1.45-1.65 (m, 11H), 1.75-1.85 (m, 2H), 3.76 (s, 3H), 3.78 (s, 2H), 3.40-3.47 (m, 2H), 3.65 (t, *J* = 7.2 Hz, 2H), 4.03 (t, *J* = 6.4 Hz, 2H), 7.01 (d, *J* = 3.2 Hz, 1H), 7.44 (dd, *J* = 8.8, 1.4 Hz, 1H), 7.75 (d, *J* = 3.2 Hz, 1H), 7.84 (d, *J* = 8.8 Hz, 1H), 7.87 (d, *J* = 1.4 Hz, 1H), 11.43 (s, 1H); ¹³C NMR(CDCl₃, 100MHz): 14.1, 19.5, 29.6, 31.6, 35.8, 36.2, 52.9, 68.5, 102.8, 116.3, 117.4, 118.1, 125.3, 129.6, 135.6, 141.0, 141.7, 143.3, 143.4, 151.6, 169.8; HRMS(ESI⁺, M+H): calc. 444.1957, found 444.1957.

### Example 11: Synthesis of Compound 2l (i.e. the compound (1-12) which is not according to the invention)

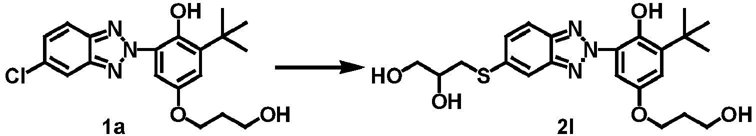

To a flask (20 mL), the compound **1a** (5.0 g), potassium carbonate (5.0 g), monothioglycerol (3.0 mL) and *N*-methylpyrrolidone (15 mL) were added, and the mixture was heated to 140°C and stirred for 4 hr. After cooling down to room temperature, the mixture was added into water (200 mL) and acidified with IN HCl aqueous solution to pH 4.5. The mixture was filtered, and the collected solids were washed with water (100 mL). The filter cake was added into isopropanol (100 mL) and heated to 60°C to dissolve the filter cake. Then, heptane (300 mL) was added into the mixture, and the temperature of the mixture was slowly cooled down. Then, water (200 mL) was added into the mixture and the temperature of the mixture was cooled down to 5°C. The precipitated solids were collected and further purified with column chromatography to obtain the compound **2l** (4.53 g, 76.0%).

¹H NMR (CDCl₃, 400MHz): 1.45 (s, 9H), 1.91 (quint, *J* = 6.4 Hz, 2H), 3.06 (dd, *J* = 7.0, 13.2 Hz, 1H), 3.20 (dd, *J* = 4.4, 13.2 Hz, 1H), 3.40-3.55 (m, 2H), 3.60 (q, *J* = 5.8 Hz, 2H), 3.70-3.82 (m, 1H), 4.07 (t*, J* = 6.6 Hz, 2H), 4.60 (t*, J* = 5.2 Hz, 1H), 4.80 (t*, J* = 5.8 Hz, 1H), 5.15 (d, *J* = 5.2 Hz, 1H), 6.94 (d, *J* = 3.0 Hz, 1H), 7.48 (dd, *J* = 1.2, 9.0 Hz, 1H), 7.59 (d, *J* = 3.0 Hz, 1H), 7.91 (d, *J* = 1.2 Hz, 1H), 7.98 (d, *J* = 9.0 Hz, 1H), 11.05 (s, 1H); ¹³C NMR (CDCl₃, 100MHz): 29.2, 32.2, 35.3, 36.1, 57.4, 64.7, 65.3, 70.1, 103.3, 112.3, 116.3, 117.8, 125.5, 129.0, 138.6, 140.2, 140.9, 142.3, 143.3, 151.0; HRMS(ESI⁺, M+H): calc. 448.1906, found 418.1913.

### Comparative example 1

The commercial available product, Eversorb^{®} 82, is represented by the following formula (II).

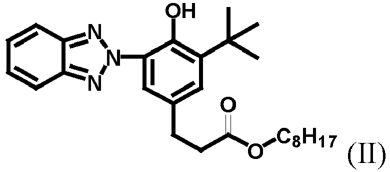

### Comparative example 2: Synthesis of Compound 6

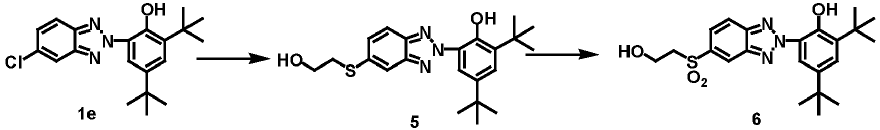

To a flask (20 mL), the compound **1e** (commercial available product, Eversorb^{®} 75) (4.0 g), KOH (1.3 g), KI (0.05 g), 2-mercaptoethanol (0.9 mL) and *N*-methylpyrrolidone (10 mL) were added, and the mixture was heated to 100°C and stirred for 6 hr. After cooling down to room temperature, the mixture was acidified with IN HCl aqueous solution to pH 5. The mixture was extracted with toluene (100 mL) and water (100 mL). The water layer was removed, and the organic layer was dried with MgSO₄ to remove water. After reduced pressure concentration and purification with column chromatography, an intermediate **5** (1.94 g, 43 %) was obtained. HRMS(ESI⁺, M+H): calc. 400.2059, found 400.2059.

To a flask (250 mL), the intermediate **5** (0.9 g), Na₂WO₄·2H₂O (0.15 g), toluene (25 mL), isopropanol (50 mL) and 30% H₂O₂ aqueous solution (15 mL) were added, and the mixture was stirred for 16 hr. The mixture was extracted with toluene (85 mL) and water (50 mL). The water layer was further extracted with toluene (75 mL) and combined with organic layer. The organic layer was washed with saturated brine (50 mL), dried with MgSO₄, and concentrated under reduced pressure to obtain the compound **6** (0.89 g, 91.6%).

¹H NMR(CDCl₃, 400MHz): 1.40 (s, 9H), 1.52 (s, 9H), 2.45 (br s, 1 H), 3.47 (t*, J* = 5.4 Hz, 2H), 4.08 (t*, J* = 5.4 Hz, 2H), 7.50 (d, *J* = 2.4 Hz, 1H), 7.94 (dd, *J* = 8.8, 1.4Hz, 1H), 8.14 (d, *J* = 8.8 Hz, 1H), 8.31 (d, *J* = 8.8 Hz, 1H), 8.69 (d, *J* = 1.4Hz, 1H), 11.39 (s, 1H); ¹³C NMR(CDCl₃, 100MHz): 29.7, 31.6, 34.8, 35.9, 56.5, 58.6, 116.6, 119.6, 120.9, 124.8, 125.0, 126.7, 138.1, 139.2, 141.7, 142.4, 144.5, 147.3; HRMS(ESI⁺, M+H): calc. 432.1957, found 432.1957.

### Comparative example 3

The commercial available product, Eversorb^{®} 78, is represented by the following formula (III).

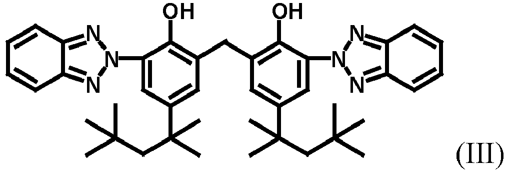

### Comparative example 4: Synthesis of Compound 7

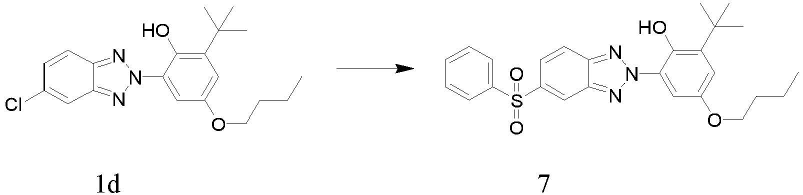

The compound **1d** (40 g) was dissolved in *N*-methylpyrrolidone (10.5 g). At 90°C, 45% KOH aqueous solution (4.43 g) was slowly added into the mixture, and then thiophenol (2.04 g) was added dropwise into the mixture. After addition, the mixture was heated to 170°C to remove water for 24 hr. Then, the mixture was cooled down to 100°C, extracted with xylene (75 mL) and washed with water (75 mL). The water layer was acidified with 15% HCl, followed by removing the water layer. The organic layer was dried with anhydrous sodium sulfate and concentrated. After placing a period of time, yellow solids were precipitated.

Next, the obtained yellow solids were dissolved in xylene (4.28 g), and sodium tungstate (0.038 g) and 90% formic acid (0.85 g) were added into the mixture. The mixture was heated to 50°C, and 30% H₂O₂ (2.54 g) was added dropwise into the mixture. The temperature of the mixture was less than 85°C during the addition. The reaction was traced with thin layer chromatography. Then, xylene and water were added into the mixture. After extraction, drying and concentraion, the product was recrystallized in methanol to obtain yellow solids, compound **7** (35.4 g, m.p. 148.3°C).

HRMS ESI [M+H]+: calc. 480.1957, found 480.1964

¹H NMR (400MHz, CDCl₃): δ 11.21 (1H), 8.71 (1H), 7.95-8.05 (3H), 7.90 (1H), 7.77 (1H), 7.40-7.70 (3H), 7.06 (1H), 4.03 (2H), 1.81 (2H), 1.54 (2H), 1.48 (9H), 1.00 (3H)

### Test example 1: Comparison of UV absorption spectra

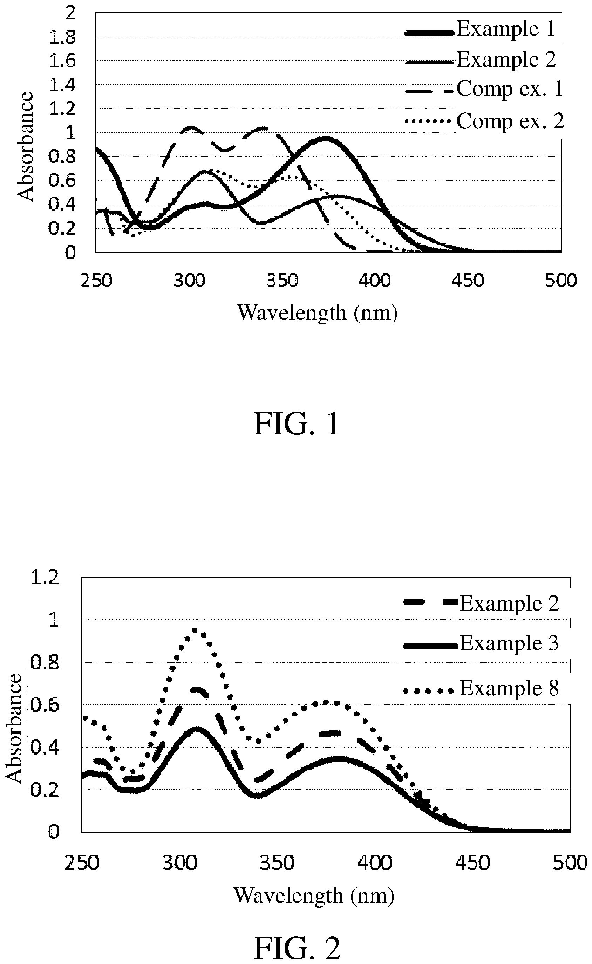

The UV absorbers of Examples 1, 2, 3 and 8 and Comparative examples 1 and 2 were formulated into 20 ppm solutions with methanol/THF (methanol : THF = 90:10). An UV/Visible spectrometer (UV-2600; Shimadzu Instruments Co., Ltd.) was used to measure the absorption of the UV absorbers. The results are shown in FIG. 1, FIG. 2 and Table 1.

**Table 1**

| Compound | Example 1 | Example 2 | Comparative example 1 | Comparative example 2 |
|---|---|---|---|---|
| Absorbance peak (nm) | 309 | 309 | 301 | 312 |
| | 373 | 379 | 340 | 355 |

As shown in the UV absorption spectra of FIG. 1, compared to the compound of Comparative example 1, the spectra of the compounds of Examples 1 and 2 show significant red shifts at the absorbance peaks. In addition, the positions of the limiting absorbance peaks of the compounds of Examples 1 and 2 have greater wavelengths than those of Comparative examples 1 and 2, and can be extended to 420 nm or more. Moreover, compared to the compound of Comparative example 2 in which no oxygen-containing group is located at the 5-position on the phenyl ring, the compound of Example 2 in which the oxygen-containing group is located at the 5-position on the phenyl ring shows a red shift at the absorbance peaks with greater wavelength (>350 nm), as shown in Table 1. These results indicate that the spectrum of the compound shows the red shift at the absorbance peaks when the sulfur-containing group is located at the 5-position on the benzo ring and the oxygen-containing group is located at the 5-position on the phenyl group.

In addition, as shown in FIG. 2, the compound of Example 2 has the feature that the oxygen atom at the 5-position on the phenyl ring is bound with the alkyl substituted with hydroxyl, and the compound of Example 3 has the feature that the oxygen atom at the 5-position on the phenyl ring is bound with the alkyl substituted with acrylate. The positions of the absorbance peaks of these two compounds are only slightly differed. Furthermore, the compound of Example 2 has the feature that the oxygen atom at the 5-position on the phenyl ring is bound with the alkyl substituted with hydroxyl, and the compound of Example 8 has the feature that the oxygen atom at the 5-position on the phenyl ring is bound with the benzyl group. The positions of the absorbance peaks of these two compounds are almost the same. These results indicate that the different reactive functional groups bound to the oxygen atoms at the 5-position on the phenyl ring do not make great influence on the absorbance peaks on the UV spectra. It is because these reactive functional groups are not directly bound to the conjugated structures of the molecules. The difference is mainly caused by the strength changes of the absorbance, which are resulted from the difference of the molecular weights and the interaction between the solvents.

### Test example 2: Transmission test

The compound of Example 2 or the compound of Comparative example 1 (0.05 g) was mixed with the polyurethane (PU) main agent (Item number: A-7121) (4 g) and ethyl acetate (2 g), followed by mixing with the curing agent (Item number: Bayer N75) (2 g). After mixing, the obtained PU glue was applied onto a PET film with a thickness of 125 µm by using a coating machine and a coating rod. After the coating process, the obtained wet film had a thickness of 100 µm. After drying at 80°C for 30 min, the transmittance of the obtained film was measured. Herein, the transmittance of the PU film was calculated to exclude the absorbance of the PET film.

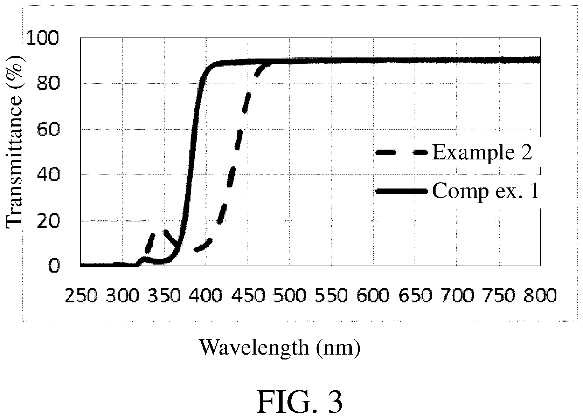

As shown in the results of FIG. 3, the spectrum of the compound of Example 2 shows the red shift, and the compound of Example 2 can absorb light having wavelengths within 400 nm to 450 nm. The results shown in FIG. 3 also indicates that the position of the limiting absorbance peak of the compound of Comparative example 1 is located at around 400 nm, but that of the compound of Example 2 is located at 450 nm or more. The aforesaid results indicate that the spectrum of the compound of Example 2 shows significant red shifts compared to the spectrum of the compound of Comparative example 1, and these results are consistent with the results measured in the solution form.

### Test example 3: Extraction test

The compound of Example 2 or the compound of Comparative example 1, 3 or 4 (0.05 g) was mixed with the polyurethane (PU) main agent (Item number: A-7121) (6 g) and the solvent (3 g), followed by mixing with the curing agent (Item number: Bayer N75) (3 g). The mixture was stirred and placed at room temperature for 4 hr, followed by drying in an oven at 80°C for 16 hr. Then, the solvent (30 g) was added, followed by extracting for 2 hr by using the ultrasonicator. After the extraction, the extraction rate was measured. Herein, the solvent for the compound of Comparative example 3 was a mixing solvent of ethyl acetate/toluene (weight ratio of ethyl acetate to toluene = 1:2), and the solvent for the rest of the compounds was ethyl acetate. The results are shown in the following Table 2.

**Table 2**

| Compound | Molecular weight | Extraction rate |
|---|---|---|
| Example 2 | 449.5 | 0%^{∗} |
| Comparative example 1 | 451.6 | 99% |
| Comparative example 3 | 658.9 | 73% |
| Comparative example 4 | 479.6 | 94% |

| | | |
|---|---|---|
| ^{∗}: 0%, which means the compound was not found. | | |

The results shown in FIG. 2 indicate that the reactive compound of Example 2 has better extraction resistance than the unreactive compound of Comparative example 1 even though these two compounds have similar molecular weight. The compound of Example 2 cannot be found after the extraction test, which means the compound of Example 2 has significant extraction resistance. However, the compound of Comparative example 1 was almost extracted out. In addition, compared to the compound of Comparative example 3 with larger molecular weight, the compound of Example 2 shows excellent extraction resistance. However, more than 70% of the compound of Comparative example 3 was extracted out. Furthermore, compared to the compound of Comparative example 4, the compound of Example 2 also shows better extraction resistance.

### Test example 4: Extraction test

The compound of Example 3 or the compound of Comparative example 1 (0.03 g) was added into a mixture of 1,6-Hexanediol diacrylate (HDDA) (0.9 g), tetrahydrofurfuryl acrylate (THFA) (1.0 g) and 1,1'-Azobis(cyclohexanecarbonitrile) (ABCN) (0.07 g). After stirring and mixing, the mixture was dried in an oven at 80°C for 24 hr. Then, ethyl acetate (10 g) was added, followed by extracting for 2 hr by using the ultrasonicator. After the extraction, the extraction rate was measured. The results are shown in the following Table 3.

**Table 3**

| Compound | Molecular weight | Extraction rate |
|---|---|---|
| Example 3 | 585.7 | 0.5% |
| Comparative example 1 | 451.6 | 93.2% |

The results shown in Table 3 indicate that the reactive compound of Example 3 has better extraction resistance than the unreactive compound of Comparative example 1. Only a small amount of the compound of Example 3 was extracted out after the extraction test, which means the compound of Example 3 has significant extraction resistance. However, the compound of Comparative example 1 was almost extracted out.

In conclusion, the spectra of the novel compounds provided by the present disclosure show red shifts at the absorbance peaks. When the novel compounds provided by the present disclosure are applied onto a substrate, which is sensitive to electromagnetic radiation with a wavelength greater than 380 nm, to form a coating layer, the obtained coating layer can effectively absorb the light having wavelength greater than 380 nm. Thus, the novel compound provided by the present disclosure can be used for forming an anti-blue light or anti-UV coating layer, to provide a product with anti-blue light or anti-UV effect, such as a protection film, glasses lens, contacts or intraocular lens. In addition, the novel compound provided by the present disclosure further has extraction resistance. When the novel compound provided by the present disclosure is polymerized with monomer or oligomer to form a polymer with UV absorbers, the application of the obtained polymer can be extended because the UV absorbers contained in the obtained polymer have excellent extraction resistance.

## Claims

1. A compound represented by the following formula (I): 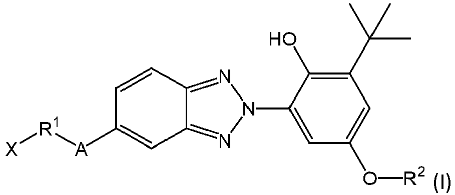 wherein,
A is -S- or -SO₂-;
R¹ is straight or branched C₁₋₁₀ alkylene;
R² is -R³Y;
R³ is straight or branched C₁₋₁₀ alkylene;
each of X and Y respectively is -OH or -OCOR⁴; and
R⁴ is straight or branched C₃₋₁₀ alkenyl.

2. The compound of claim 1, wherein R⁴ is straight or branched C₃₋₆ alkenyl.

3. The compound of claim 1, wherein A is -S-; R¹ is straight or branched C₁₋₁₀ alkylene; and X is -OH.

4. The compound of claim 3, wherein R¹ is straight or branched C₁₋₆ alkylene.

5. The compound of claim 1, wherein A is -SO₂-; R¹ is straight or branched C₁₋₁₀ alkylene; X is -OH or -OCOR⁴; and R⁴ is straight or branched C₃₋₁₀ alkenyl; preferably
wherein R¹ is straight or branched C₁₋₆ alkylene; X is -OH or -OCOR⁴; and
R⁴ is straight or branched C₃₋₆ alkenyl.

6. The compound of claim 1, wherein R² is -R³Y;
wherein R³ is straight or branched C₁₋₆ alkylene; Y is -OH or -OCOR⁴; and
R⁴ is straight or branched C₃₋₆ alkenyl.

7. The compound of claim 1, which is any one of the following formulas (I-1) to (I-3):

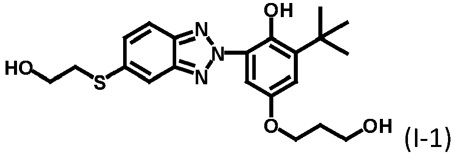

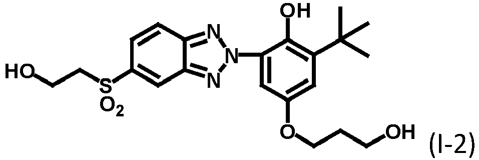

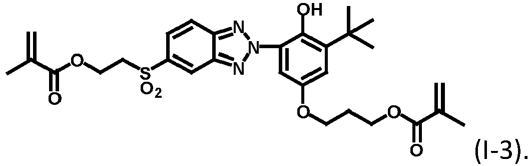

8. A composition with stability to photo-induced degradation, including:
(A) a photo-induced degradable organic material; and
(B) a compound represented by the following formula (I): 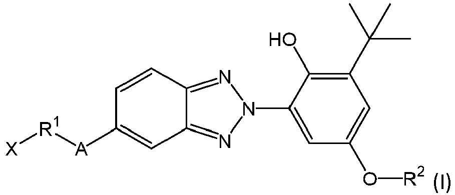 wherein,
A is -S- or -SO₂-;
R¹ is straight or branched C₁₋₁₀ alkylene;
R² is -R³Y;
R³ is straight or branched C₁₋₁₀ alkylene;
each of X and Y respectively is -OH or -OCOR⁴; and
R⁴ is straight or branched C₃₋₁₀ alkenyl.

9. The composition of claim 8, which is used for forming a coating layer.

10. The composition of claim 9, wherein the coating layer is formed on a substrate which is sensitive to electromagnetic radiation with a wavelength greater than 380 nm.

11. The composition of any of the claims 8 to 10, wherein a content of the compound is 0.1% to 30% based on a weight of the photo-induced degradable organic material.

12. A glasses lens with anti-blue light effect, comprising a compound represented by the following formula (I): 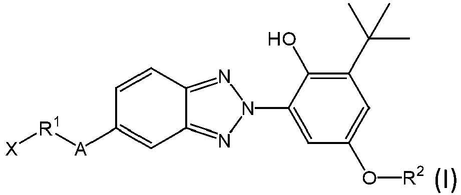 wherein,
A is -S- or -SO₂-;
R¹ is straight or branched C₁₋₁₀ alkylene;
R² is -R³Y;
R³ is straight or branched C₁₋₁₀ alkylene;
each of X and Y respectively is -OH or -OCOR⁴; and
R⁴ is straight or branched C₃₋₁₀ alkenyl.

13. A protection film with anti-blue light effect, comprising a compound represented by the following formula (I): 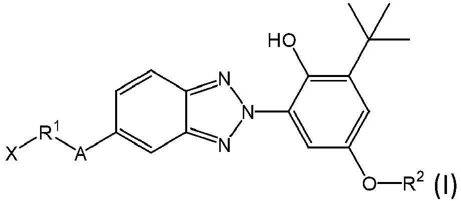 wherein,
A is -S- or -SO₂-;
R¹ is straight or branched C₁₋₁₀ alkylene;
R² is -R³Y;
R³ is straight or branched C₁₋₁₀ alkylene;
each of X and Y respectively is -OH or -OCOR⁴; and
R⁴ is straight or branched C₃₋₁₀ alkenyl.

## Patentansprüche

1. Verbindung, dargestellt durch die folgende Formel (I) 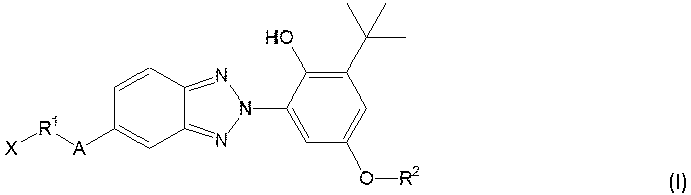 worin,
A -S- oder -SO₂- ist;
R¹ ein geradkettiges oder verzweigtes C₁₋₁₀-Alkylen ist;
R² -R³Y ist;
R³ ein geradkettiges oder verzweigtes C₁₋₁₀-Alkylen ist;
X und Y sind jeweils -OH oder -OCOR⁴; und
R⁴ ein geradkettiges oder verzweigtes C₃₋₁₀-Alkenyl ist.

2. Verbindung nach Anspruch 1, wobei R⁴ ein geradkettiges oder verzweigtes C₃₋₆-Alkenyl ist.

3. Verbindung nach Anspruch 1, worin A -S- ist; R¹ ein geradkettiges oder verzweigtes C₁₋₁₀-Alkylen ist; und X -OH ist.

4. Verbindung nach Anspruch 3, wobei R¹ ein geradkettiges oder verzweigtes C₁₋₆-Alkylen ist.

5. Verbindung nach Anspruch 1, worin A -SO₂- ist; R1 ein geradkettiges oder verzweigtes C₁₋₁₀-Alkylen ist; X -OH oder -OCOR⁴ ist; und R⁴ ein geradkettiges oder verzweigtes C₃₋₁₀-Alkenyl ist; vorzugsweise worin R¹ ein geradkettiges oder verzweigtes C₁₋₆-Alkylen ist; X -OH oder -OCOR⁴ ist; und R⁴ ein geradkettiges oder verzweigtes C₃₋₆-Alkenyl ist.

6. Verbindung nach Anspruch 1, worin R² -R³Y ist, wobei R³ ein geradkettiges oder verzweigtes C₁₋₆-Alkylen ist; Y -OH oder -OCOR⁴ ist; und R⁴ ein geradkettiges oder verzweigtes C₃₋₆-Alkenyl ist.

7. Die Verbindung nach Anspruch 1, die eine der folgenden Formeln (I-1) bis (I-3) aufweist:

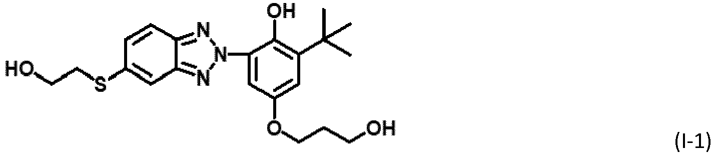

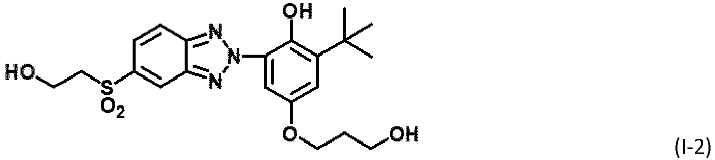

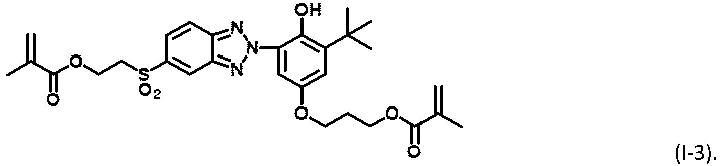

8. Zusammensetzung, die gegenüber photo-induziertem Abbau stabil ist, umfassend:
(A) ein photoinduziert abbaubares organisches Material; und
(B) eine Verbindung, dargestellt durch die folgende Formel (I): 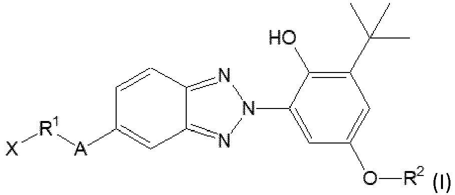 worin,
A -S- oder -SO₂- ist;
R1 ein geradkettiges oder verzweigtes C₁₋₁₀-Alkylen ist;
R² -R³Y ist;
R³ ein geradkettiges oder verzweigtes C₁₋₁₀-Alkylen ist;
X und Y jeweils -OH oder -OCOR⁴ sind; und
R⁴ ein geradkettiges oder verzweigtes C₃₋₁₀-Alkenyl ist.

9. Zusammensetzung nach Anspruch 8, die zur Bildung einer Überzugsschicht verwendet wird.

10. Zusammensetzung nach Anspruch 9, wobei die Überzugsschicht auf einem Substrat gebildet wird, das für elektromagnetische Strahlung mit einer Wellenlänge von mehr als 380 nm empfindlich ist.

11. Zusammensetzung nach einem der Ansprüche 8 bis 10, wobei der Gehalt an der Verbindung 0,1 % bis 30 %, bezogen auf das Gewicht des photoinduziert abbaubaren organischen Materials, beträgt.

12. Brillenglas mit Anti-Blaulicht-Effekt, umfassend eine Verbindung der folgenden Formel (I): 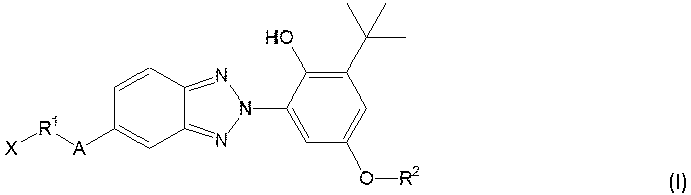 worin,
A -S- oder -SO₂- ist;
R1 ein geradkettiges oder verzweigtes C₁₋₁₀-Alkylen ist;
R2 -R³Y;
R³ ein geradkettiges oder verzweigtes C₁₋₁₀-Alkylen ist;
X und Y jeweils -OH oder -OCOR⁴ sind; und
R⁴ ein geradkettiges oder verzweigtes C₃₋₁₀-Alkenyl ist.

13. Schutzfilm mit Anti-Blaulicht-Effekt, umfassend eine Verbindung der folgenden Formel (I): 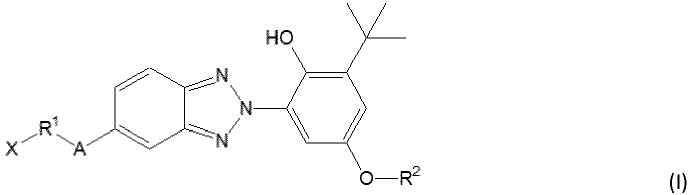 worin,
A -S- oder -SO₂- ist;
R¹ ein geradkettiges oder verzweigtes C₁₋₁₀-Alkylen ist;
R² -R³Y ist;
R³ ein geradkettiges oder verzweigtes C₁₋₁₀-Alkylen ist;
X und Y jeweils -OH oder -OCOR⁴ sind; und
R⁴ ein geradkettiges oder verzweigtes C₃₋₁₀-Alkenyl ist.

## Revendications

1. Composé représenté par la formule (I) suivante : 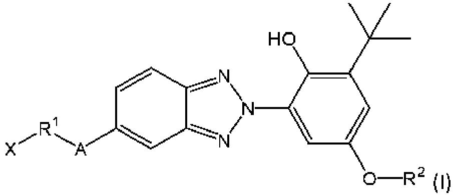 dans laquelle :
A représente -S- ou -SO₂- ;
R¹ représente un groupe alkylène en C₁₋₁₀ linéaire ou ramifié :
R² représente -R³Y ;
R³ représente un groupe alkylène en C₁₋₁₀ linéaire ou ramifié ;
chacun de X et Y représente respectivement -OH ou -OCOR⁴ et
R⁴ représente un groupe alcényle en C₃₋₁₀ linéaire ou ramifié.

2. Composé selon la revendication 1, dans lequel R⁴ représente un groupe alcényle en C₃₋₆ linéaire ou ramifié.

3. Composé selon la revendication 1, dans lequel A représente -S- ; R¹ représente un groupe alkylène en C₁₋₁₀ linéaire ou ramifié ; et X représente -OH.

4. Composé selon la revendication 3, dans lequel R¹ représente un groupe alkylène en C₁₋₆ linéaire ou ramifié.

5. Composé selon la revendication 1, dans lequel A représente -SO₂- ; R¹ représente un groupe alkylène en C₁₋₁₀ linéaire ou ramifié ; X représente -OH ou -OCOR⁴ ; et R⁴ représente un groupe alcényle en C₃₋₁₀ linéaire ou ramifié ; de préférence
dans lequel R¹ représente un groupe alkylène en C₁₋₆ linéaire ou ramifié ; X représente -OH ou -OCOR⁴ ; et R⁴ représente un groupe alcényle en C₃₋₆ linéaire ou ramifié.

6. Composé selon la revendication 1, dans lequel R² représente -R³Y ;
dans lequel R³ représente un groupe alkylène en C₁₋₆ linéaire ou ramifié ; Y représente -OH ou -OCOR⁴ ; et R⁴ représente un groupe alcényle en C₃₋₆ linéaire ou ramifié.

7. Composé selon la revendication 1, qui est l'une quelconque des formules (I-1) à (1-3) suivantes :

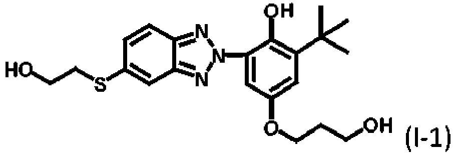

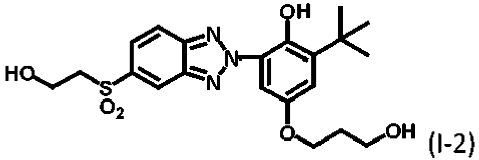

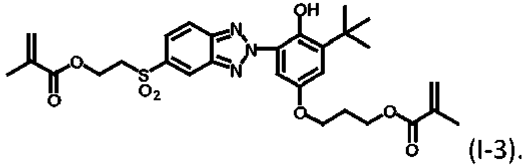

8. Composition présentant une stabilité à la dégradation photo-induite, comprenant :
(A) une matière organique dégradable de façon photo-induite ; et
(B) un composé représenté par la formule (I) suivante : 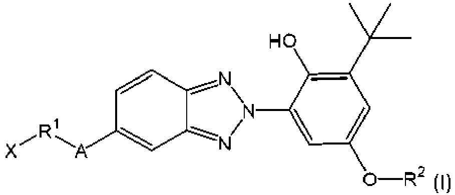 dans laquelle :
A représente -S- ou -SO₂- ;
R¹ représente un groupe alkylène en C₁₋₁₀ linéaire ou ramifié ;
R² représente -R³Y ;
R³ représente un groupe alkylène en C₁₋₁₀ linéaire ou ramifié ;
chacun de X et Y représente respectivement -OH ou -OCOR⁴ ; et
R⁴ représente un groupe alcényle en C₃₋₁₀ linéaire ou ramifié.

9. Composition selon la revendication 8, qui est utilisée pour former une couche de revêtement.

10. Composition selon la revendication 9, dans laquelle la couche de revêtement est formée sur un substrat qui est sensible au rayonnement électromagnétique d'une longueur d'onde supérieure à 380 nm.

11. Composition selon l'une quelconque des revendications 8 à 10, dans laquelle la teneur en composé est de 0,1 % à 30 % sur la base du poids de la matière organique dégradable de façon photo-induite.

12. Verre de lunettes à effet anti-lumière bleue, comprenant un composé représenté par la formule (I) suivante : 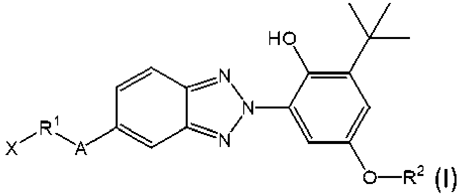 dans laquelle :
A représente -S- ou -SO₂- ;
R¹ représente un groupe alkylène en C₁₋₁₀ linéaire ou ramifié ;
R² représente -R³Y ;
R³ représente un groupe alkylène en C₁₋₁₀ linéaire ou ramifié ;
chacun de X et Y représente respectivement -OH ou -OCOR⁴ ; et
R⁴ représente un groupe alcényle en C₃₋₁₀ linéaire ou ramifié.

13. Film de protection à effet anti-lumière bleue, comprenant un composé représenté par la formule (I) suivante : 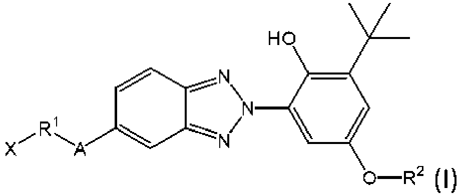 dans laquelle :
A représente -S- ou -SO₂- ;
R¹ représente un groupe alkylène en C₁₋₁₀ linéaire ou ramifié ;
R² représente -R³Y ;
R³ représente un groupe alkylène en C₁₋₁₀ linéaire ou ramifié ;
chacun de X et Y représente respectivement -OH ou -OCOR⁴ ; et
R⁴ représente un groupe alcényle en C₃₋₁₀ linéaire ou ramifié.
